# EUROPÄISCHE PATENTSCHRIFT

(11) **EP 1 845 936 B1**
(45) Veröffentlichungstag und Bekanntmachung des Hinweises auf die Patenterteilung: **01.12.2010**
(21) Anmeldenummer: 06706449.3
(22) Anmeldetag: 27.01.2006
(51) Int. Cl.: A61K 8/97, A61Q 5/00, A61Q 11/00, A61Q 19/00

(54) **ROOIBOS-EXTRAKT MIT ERHÖHTEM ASPALATHINGEHALT UND VERFAHREN ZUR HERSTELLUNG EINES SOLCHEN ROOIBOS-EXTRAKTES**
ROOIBOS EXTRACT WITH AN INCREASED ASPALATHIN CONTENT AND METHOD FOR PRODUCING ONE SUCH ROOIBOS EXTRACT
EXTRAIT DE ROOIBOS A TENEUR EN ASPALTHINE ELEVEE ET PROCEDE DE PRODUCTION DUDIT EXTRAIT

(30) Priorität: 31.01.2005 DE 102005004438
(43) Veröffentlichungstag der Anmeldung: 24.10.2007
(73) Patentinhaber: Raps GmbH & Co. KG, 95326 Kulmbach (DE)
(72) Erfinder: GRÜNER-RICHTER, Sabine, 85354 Freising (DE); OTTO, Frank, 85402 Kranzberg (DE); WEINREICH, Bernd, 82377 Penzberg (DE)
(74) Vertreter: Beyer, Andreas
(86) Internationale Anmeldenummer: PCT/EP2006/000723
(87) Internationale Veröffentlichungsnummer: WO 2006/081989

(56) Entgegenhaltungen:
- WO-A-2005/041854
- SCHULZ HARTWIG ET AL: "Quantification of quality parameters for reliable evaluation of green rooibos (Aspalathus linearis)." EUROPEAN FOOD RESEARCH AND TECHNOLOGY, Bd. 216, Nr. 6, Juni 2003 (2003-06), Seiten 539-543, XP002375308 ISSN: 1438-2377
- BRAMATI LORENZO ET AL: "Unfermented rooibos tea: Quantitative characterization of flavonoids by HPLC-UV and determination of the total antioxidant activity." JOURNAL OF AGRICULTURAL AND FOOD CHEMISTRY, Bd. 51, Nr. 25, 3. Dezember 2003 (2003-12-03), Seiten 7472-7474, XP002375309 ISSN: 0021-8561
- JOUBERT ELIZABETH ET AL: "Superoxide anion and alpha, alpha-diphenyl-beta-picrylhydrazyl radical scavenging capacity of rooibos (Aspalathus linearis) aqueous extracts, crude phenolic fractions, tannin and flavonoids." FOOD RESEARCH INTERNATIONAL, Bd. 37, Nr. 2, 2004, Seiten 133-138, XP002375310 ISSN: 0963-9969
- SCHULZ H: "RED BUSH IN COSMETICS" COSSMA: COSMETICS, SPRAY TECHNOLOGY, MARKETING, BRAUN FACHVERLAGE, KARLSRUHE, DE, Bd. 1, Nr. 4, April 2000 (2000-04), Seiten 22-23, XP009033110 ISSN: 1439-7676
- AHN T U ET AL: "EXTRACTING PROCESS FOR EFFECTIVE INGREDIENT FROM ROOIBOS, AND PREPARING PROCESS FOR COSMETIC WATER, WEAK-OILY CREAM AND EMOLLIENT CREAM USING ROOIBOS EXTRACT" 4. August 2003 (2003-08-04), WPI DERWENT , XP002985215 Zusammenfassung
- AHN T U: "METHOD OF EXTRACTING ANTIOXIDANT FROM ROOIBOS TEA AND PRODUCTION OF COSMETIC ROOIBOS TEA EXTRACT" 29. März 2003 (2003-03-29), WPI DERWENT , XP002985214 Zusammenfassung
- PATENT ABSTRACTS OF JAPAN Bd. 1999, Nr. 09, 30. Juli 1999 (1999-07-30) & JP 11 116429 A (MIKIMOTO PHARMACEUT CO LTD; TOYO HAKKO:KK), 27. April 1999 (1999-04-27)

## Beschreibung

Die vorliegende Erfindung betrifft einen neuartigen Rooibos-Extrakt und ein Verfahren zur Herstellung eines solchen Rooibos-Extraktes. Vorzugsweise hat der erfindungsgemäße Rooibos-Extrakt auch einen stark erniedrigten Chlorophyllgehalt.

Der Rooibos, deutsch Rotbusch, lateinisch Aspalathus linearis, wächst ausschließlich in Südafrika und enthält als weltweit einzige Pflanze die besonders stark antioxidativ wirkende Substanz Aspalathin, ein Flavonoid. Vielen Leuten ist der Rooibos vom Rotbuschtee her bekannt, denn fermentierte und getrocknete Rooibos-Blätter werden traditionell als Tee oder Teeextrakt verwendet. Die Rooibos-Pflanze ist alkaloidfrei, enthält insbesondere kein Koffein und weist im Vergleich zu z.B. grünem Tee nur einen geringen Gerbstoffgehalt auf, was Rotbuschtee verträglich auch für magenempfindliche Personen macht.

Kommerziell erhältlich sind Rotbuschtee-Extrakte, die einen Aspalathingehalt von nicht mehr als 1 bis 3 % aufweisen. Zur besseren Nutzung der erwähnten antioxidativen Wirkung des im Rooibos enthaltenen Aspalathins wäre es wünschenswert, einen Rooibos-Extrakt mit einem höheren Aspalathingehalt einsetzen zu können. Zugleich wäre es wünschenswert, dass ein solcher Extrakt einen möglichst geringen Chlorophyllgehalt aufweist, da ein Rooibos-Extrakt mit einem hohen Chlorophyllgehalt bei der Verwendung in kosmetischen Mitteln zu einer unerwünschten Färbung derselben führt.

H. Schulz et al. beschreiben in einem Artikel mit dem Titel "Quantification of quality parameters for reliable evaluation of green rooibos (Aspalathus linearis)" in der Zeitschrift EUROPEAN FOOD RESEARCH AND TECHNOLOGY, Bd. 216, Nr. 6, Juni 2003, Seiten 539-543 die Herstellung eines Rooibos-Extrakts zur Verwendung in der HPLC-Analyse. L. Bramati et al. beschreiben in einem Artikel mit dem Titel "Unfermented Rooibos Tea: Quantitative Characterization of Flavonoids by HPLC-UV and Determination of the Total Antioxidant Activity" in der Zeitschrift JOURNAL OF AGRICULTURAL AND FOOD CHEMISTRY, Bd. 51, Nr. 25, 3. Dezember 2003, Seiten 7472-7474 ein Herstellungsverfahren für einen Rooibos-Extrakt. Ein weiteres Herstellungsverfahren für einen Rooibos-Extrakt beschreiben E. Joubert et al. in einem Artikel mit dem Titel "Superoxide anion and α-α-diphenyl-β-picrylhydrazyl radical scavenging capacity of rooibos (Aspalathus linearis) aqueous extracts, crude phenolic fractions, tannin and flavonoids" in der Zeitschrift FOOD RESEARCH INTERNATIONAL, Bd. 37, Nr. 2, 2004, Seiten 133-138. Alle vorgenannten, aus dem Stand der Technik bekannten Herstellungsverfahren für Rooibos-Extrakte basieren auf einer Extraktion mit Wasser.

Der Erfindung liegt die Aufgabe zugrunde, einen Rooibos-Extrakt mit erhöhtem Aspalathingehalt und vorzugsweise deutlich verringertem Chlorophyllgehalt bereitzustellen und ein Verfahren zur Herstellung eines solchen verbesserten Rooibos-Extraktes anzugeben, so dass ein solcher Rooibos-Extrakt auch in kosmetischen Mitteln Verwendung finden kann.

Diese Aufgabe ist erfindungsgemäß gelöst durch einen mittels Lösemittelextraktion aus unfermentierter Rooibos-Rohware hergestellten Rooibos-Extrakt, der einen Aspalathingehalt von mindestens 10 Gew.-% und einen Chlorophyllgehalt von weniger als 0,4 Gew.-% aufweist, wobei die Lösemittelextraktion die im Anspruch 1 angegebenen Schritte umfasst. Ein Aspalathingehalt von mindestens 10 Gew.-% ist ausreichend hoch, um bei einer Anwendung des Extraktes in z.B. kosmetischen Mitteln eine medizinische Wirksamkeit zu gewährleisten. Der Chlorophyllgehalt von weniger als 0,4 Gew.-%, vorzugsweise von weniger als 0,2 Gew.-%, und besonders vorzugsweise von weniger als 0,1 Gew.-% verhindert eine unerwünschte Färbung eines mit dem erfindungsgemäßen Rooibos-Extrakt versehenen Produktes.

Vorzugsweise beträgt der Aspalathingehalt des erfindungsgemäßen Rooibos-Extraktes mehr als 15 Gew.-% oder sogar mehr als 20 Gew.-%. Rooibos-Extrakte mit solch hohen Gehalten an Aspalathin waren bisher nicht erhältlich. Der Vorteil eines hohen Aspalathingehalts ist darin zu sehen, dass bei einem gegebenen Produkt zur Erzielung gleicher Wirksamkeit die Zugabe einer geringeren Rooibos-Extraktmenge ausreicht, was aufgrund des dann ebenfalls niedrigeren Chlorophyllgehaltes auch die Gefahr einer unerwünschten Verfärbung des Produktes verringert. Ein weiterer Vorteil eines hohen Aspalathingehalts liegt darin begründet, dass mit einer gegebenen Menge an Rooibos-Extrakt, die einem Produkt zugeführt wird, der Gehalt an kosmetisch wirksamem Aspalathin deutlich erhöht ist. Aspalathin wirkt nicht nur stark antioxidativ, sondern auch antiirritativ und antimikrobiell, was insbesondere für einen Einsatz des erfindungsgemäßen Extraktes in kosmetischen Mitteln zur Haar-, Haut- oder Mundpflege oder -behandlung von Vorteil ist.

Vorzugweise enthält der erfindungsgemäße Rooibos-Extrakt noch geringere Chlorophyllgehalte als zuvor angegeben, besonders bevorzugt weniger als 0,15 Gew.-%. Der erfindungsgemäße Rooibos-Extrakt eignet sich, wie bereits erwähnt, besonders gut zur Verwendung in kosmetischen Mitteln zur Behandlung oder Pflege der Haut, der Haare oder auch des Mundraumes. Ein solches kosmetisches Mittel liegt vorzugsweise in Form einer Lösung, einer Emulsion oder in Form eines Gels vor. Beispiele für solche kosmetische Mittel sind ein Zahngel, eine After-Sun-Lotion, eine getönte Tagescreme oder auch ein Lippenstift.

Wenn ein den erfindungsgemäßen Rooibos-Extrakt enthaltendes kosmetisches Mittel als Emulsion vorliegt, ist diese Emulsion vorzugsweise eine Creme. Wird eine für kosmetische Mittel übliche Cremegrundlage mit 0,1 Gew.-% des erfindungsgemäßen Rooibos-Extraktes versehen, so zeigt die sich ergebende Creme eine nachweislich antiirritative Wirkung im sogenannten Halbseitentest und senkt bei mehrtägiger Applikation auf die Haut den transepidermalen Wasserverlust (TEWL = transepidermal water loss) deutlich. Des Weiteren zeigt eine solche mit dem erfindungsgemäßen Extrakt versehene Creme eine regenerationsunterstützende Wirkung auf die Haut.

Neben der stark antioxidativen Wirkung des erfindungsgemäßen Extraktes, die durch wissenschaftlich anerkannte Tests wie ABTS, Fenton, Peroxynitrid, Rose Bengal, kupferinduziertes Rose Bengal, Xanthin/Xanthinoxidase bestätigt worden ist, zeigt der erfindungsgemäße Rooibos-Extrakt auch eine antimikrobielle Wirkung, z.B. auf Keime von Escherichia Coli, Staphylococcus Aureus, Pseudomomas Aeroginosa sowie ferner eine fungizide Wirkung beispielsweise hinsichtlich Aspergillus Niger.

Um kosmetische Mittel, die den erfindungsgemäßen Rooibos-Extrakt enthalten, auch über längere Zeit vor einer unerwünschten Verfärbung zu schützen, enthalten diese kosmetischen Mittel vorzugsweise 0,01 bis 2 Gew.-%, insbesondere 0,1 bis 2 Gew.-% eines vor Verfärbung schützenden Additivs. Beispiele für ein solches Additiv sind Ascorbinsäure, Ascorbylpalmitat, α-Tocoperhol, α-Tocopherylacetat, tert.-Butyl-Hydrochinon, Hydrochinon, Propylgallat, Na-Chlorat, Na-EDTA, Gallussäure, Phytinsäure, Rosmarinsäure, ein carnosolsäurehaltiger Extrakt aus Rosmarin oder Mischungen dieser Stoffe.

Alternativ besteht die Möglichkeit kosmetische Mittel, die den erfindungsgemäßen Rooibos-Extrakt enthalten, durch eine Absenkung des pH-Wertes auf 4 oder kleiner vor Verfärbung zu schützen.

Die Herstellung eines erfindungsgemäßen Rooibos-Extraktes mit erhöhtem Aspalathingehalt und vorzugsweise auch erniedrigtem Chlorophyllgehalt gelingt besonders gut mit einem speziellen Verfahren, welches deshalb ebenfalls Gegenstand der vorliegenden Erfindung ist. Demgemäß wird die eingangs genannte Aufgabe auch gelöst durch ein Verfahren zur Herstellung eines erfindungsgemäßen Rooibos-Extraktes, das folgende Schritte aufweist:
- Bereitstellen getrockneter und zerkleinerter, unfermentierter Rooibos-Rohware,
- Extrahieren der bereitgestellten Rohware mit einem Extraktionsmittel bestehend aus einer Mischung aus Ethanol und Wasser für eine vorbestimmte Extraktionsdauer bei einer Temperatur von 15 bis 35 °C,
- Filtrieren des Extraktes,
- Einengen des filtrierten Extraktes unter reduziertem Druck bis zur Trockene,
- Mahlen des getrocknetes Extraktes,
- Waschen des gemahlenen Extraktes mit einem Lösemittel für eine vorbestimmte Zeitdauer,
- Abfiltrieren des Extrakt/Lösungsmittel-Gemisches und Trocknen des resultierenden Filterkuchens unter reduziertem Druck bis zum Erreichen eines vorgegebenen Restgehaltes and Lösemittel.

Wichtig für das erfindungsgemäße Verfahren ist demnach das Bereitstellen eines geeigneten Ausgangsmaterials, das aus getrockneter und unfermentierter Rooibos-Rohware besteht. Üblicherweise ist das Ausgangsmaterial eine bei der Ernte anfallende Mischung aus Blättern und Stängeln der *Aspalathus linearis*-Pflanze, jedoch können mit Vorteil auch nur Blätter als Ausgangsmaterial dienen. Vorzugsweise ist der Feuchtegehalt der bereitgestellten Rooibos-Rohware 4 % oder kleiner, da auf diese Weise eine Autofermentation des Ausgangsmaterials verhindert ist. Wenn nur Blätter als Ausgangsmaterial verwendet werden, braucht der Feuchtegehalt der bereitgestellten Rooibos-Rohware lediglich 7 % oder weniger zu betragen. Ein Feuchtegehalt von 7 % entspricht in diesem Fall (nur Blätter als Ausgangsmaterial) einem Feuchtegehalt von 4 % bei einer Mischung von Blättern und Stängeln, da die Stängel sich besser trocknen lassen und deshalb im Falle einer Mischung zu einer geringeren Durchschnittsfeuchte der getrockneten Mischung führen, d.h. die Stängel haben in der Mischung gegenüber den Blättern einen deutlich niedrigeren Feuchtegehalt.

Als Extraktionsmittel wird gemäß einer bevorzugten Ausgestaltung des erfindungsgemäßen Verfahrens eine Ethanol/Wasser-Mischung im Verhältnis 80 zu 20 verwendet. Bei dieser bevorzugten Ausführungsform des erfindungsgemäßen Verfahrens beträgt das Verhältnis von Rohware zu Extraktionsmittel vorzugsweise 1 zu 5 und der Schritt des Extrahierens erfolgt vorzugsweise bei Raumtemperatur für eine Dauer von 1,5 bis 3 Stunden. Es können jedoch auch andere Ethanol/Wasser-Mischungsverhältnisse zum Einsatz kommen, bei denen der Ethanolanteil höher, aber auch niedriger, unter Umständen deutlich niedriger sein kann.

Der Schritt des Einengens des filtrierten Extraktes erfolgt vorzugsweise bei einem Druck von weniger als 100 mbar, und insbesondere bei einem Druck von weniger als 30 mbar. Die Temperatur beträgt bei dem Einengungsschritt vorzugsweise maximal 40°C.

Der Schritt des Waschens des gemahlenen Extraktes mit einem Lösemittel erfolgt vorzugsweise bei einem Verhältnis von Extrakt zu Lösemittel von 1 zu 5 für eine Zeitdauer von 1,5 bis 3 Stunden.

Der vorgegebene Restgehalt an Lösemittel, bis zu dessen Erreichen der Filterkuchen aus Extrakt/Lösemittel-Gemisch getrocknet wird, beträgt vorzugsweise 20 ppm oder weniger.

Eine besonders bevorzugte Ausgestaltung des erfindungsgemäßen Verfahrens zur Herstellung des erfindungsgemäßen Rooibos-Extraktes wird im Folgenden näher erläutert. Als Ausgangsmaterial wird schonend auf einen Feuchtegehalt von kleiner als 4 % getrocknete, unfermentierte und zerkleinerte Rohware (Blätter und Stängel) von Aspalathus linearis verwendet. Dieses Rohmaterial wird mit einer Mischung aus Ethanol und Wasser im Verhältnis 80 zu 20 bei 15 bis 35 °C für 1,5 bis 3 Stunden extrahiert, wobei das Verhältnis Rohware zu Lösemittel 1 zu 5 beträgt.

Der erhaltene Extrakt wird filtriert und unter reduziertem Druck, vorzugsweise bei 30 mbar und bei einer Temperatur von maximal 40°C bis zur Trockene eingeengt. Der getrocknete Extrakt wird anschließend zermahlen und mit Ethylacetat für eine Dauer von 1,5 bis 3 Stunden bei Raumtemperatur gewaschen, wobei das Verhältnis Extrakt zu Löse- bzw. Waschmittel 1 zu 5 beträgt.

Die Mischung aus Ethylacetat und dem damit gewaschenen Extrakt wird nach dem Waschvorgang filtriert, das Filtrat sodann verworfen und der Filterkuchen unter reduziertem Druck solange getrocknet, bis der messbare Lösemittelrückstand im Filterkuchen kleiner als 20 ppm ist.

Man erhält ein feines, beige-grünes Pulver mit einem Aspalathingehalt von mindestens 15 Gew.-%, wobei der erhaltene Extrakt nur 1 bis maximal 3 % des Chlorophylls der Rohware enthält.

Im Folgenden wird ein Herstellungsbeispiel angegeben:
4 Kilogramm einer getrockneten und zerkleinerten Rooibos-Rohware (Blätter und Stängel) mit einem Gehalt von 3,7 % Aspalathin werden mit einer Mischung aus 16 Kilogramm Ethanol und 4 Kilogramm Wasser 90 Minuten lang bei 30°C extrahiert.

Nach dem Abfiltrieren des unlöslichen Rückstandes von Aspalathus linearis wird das erhaltene Filtrat bei einem reduzierten Druck von unter 100 mbar und einer Temperatur von 40°C bis zur Trockene eingeengt. Dieser Rohextrakt wird fein vermahlen, mit 1.800 Gramm Ethylacetat versetzt und für die Dauer von 90 Minuten bei Raumtemperatur gerührt. Anschließend wird die Mischung abfiltriert und der Filterkuchen unter reduziertem Druck von weniger als 100 mbar für die Dauer von 2 Stunden getrocknet. Man erhält 340 Gramm Extrakt mit einem Gehalt von 16,2 Gewichtsprozent Aspalathin. Der Extrakt enthält 0,058 Gewichtsprozent Chlorophyll, das entspricht 0,9 % des Chlorophyllgehalts der verwendeten Rohware.

Ein gemäß dem zuvor erläuterten Verfahren hergestellter, erfindungsgemäßer Rooibos-Extrakt wurde in verschiedenen kosmetischen Mitteln gemäß den folgenden Beispielen eingesetzt.

### Beispiel 1: Zahngel mit folgender Zusammensetzung

| Handelsname | CTFA | INCI | Anteil in % |
|---|---|---|---|
| Sorbitol | Sorbitol | Sorbitol | 40,000 |
| Sident S 9 | Silica | Silica | 12,000 |
| Glycerin | Glycerin | Glycerin | 10,000 |
| Sident S 22 | Silica | Silica | 10,000 |
| Tagat L 2 | PEG-20 Glyceryl Laurate | PEG-20 Glyceryl Laurate | 5,000 |
| Extrapon Salbei GW | | | |
| | Glycerin / Aqua / | Glycerin / Aqua / | |
| | Sage Extract / | Sage Extract / | |
| | Sodium Benzoate / | Sodium Benzoate / | |
| | Potassium Sorbate | Potassium Sorbate | 1,000 |
| Extrapon Kamille GW | | | |
| | Glycerin / Aqua / | Glycerin / Aqua / | |
| | Matricaria Extract / | Matricaria Extract / | |
| | Lactic Acid / Sodium | Lactic Acid / Sodium | |
| | Benzoate / Potassium | Benzoate / Potassium | |
| | Sorbate | Sorbate | 1,000 |
| Rooibos | Rooibus (Leaf) Extract | Aspalathus Linearis | 0,200 |
| Natrium-Saccharin | Sodium Saccharinate | Sodium Saccharinate | 0,100 |
| Blanose 7 M F | Cellulose Gum | Cellulose Gum | 0,100 |
| Wasser, Konservierung, Aroma | | | ad 100 |

### Beispiel 2: After-Sun-Lotion mit folgender Zusammensetzung

| Handelsname | CTFA | INCI | Anteil in % |
|---|---|---|---|
| Miglyol 812 | Caprylic/Capric- | Caprylic/Capric- | |
| | Triglyceride | Triglyceride | 6,000 |
| Alkohol, verg. | SD-Alcohol 39 C | Alcohol denat. | 5,000 |
| Propylenglykol | Propylene Glykol | Propylene Glykol | 5,000 |
| Cetiol SN | Cetearyl Isononanoate | Cetearyl Isononanoate | 4,000 |
| Cetiol V | Decyl Oleate | Decyl Oleate | 2,500 |
| Tego Care 450 | Polyglyceryl-3- | Polyglyceryl-3- | |
| | Methylglucose Distearat | Methylglucose Distearat | 2,250 |
| Abil 100 | Dimethicone | Dimethicone | 0,500 |
| Pemulen TR 1 | Acrylates/C10-30 Alkyl | Acrylates/C10-30 Alkyl | |
| | Acrylate Crosspolymer | Acrylate Crosspolymer | 0,200 |
| Rooibos | Rooibus (Leaf) Extract | Aspalathus Linearis | 0,150 |
| Natriumhyroxid- | Sodium Hydroxide | Sodium Hydroxide | 0,020 |
| Plätzchen | | | |
| Wasser, Konservierung, PÖ | | | ad 100 |

### Beispiel 3: Getönte Tagescreme mit folgender Zusammensetzung

| Handelsname | CTFA | INCI | Anteil in % |
|---|---|---|---|
| Arlatone 983 | PEG-5 Glyceryl Stearate | PEG-5 Glyceryl Stearate | 4,000 |
| Stearinsäure | Stearic Acid | Stearic Acid | 0,500 |
| Tegin SE | Glyceryl Stearate SE | Glyceryl Stearate SE | 1,200 |
| Lanette 16 | Cetyl Alcohol | Cetyl Alcohol | 2,000 |
| Abil 350 | Dimethicone | Dimethicone | 1,000 |
| Tegosoft OS | Octyl Stearate | Octyl Stearate | 3,000 |
| Phenova | Phenoxyethanol / | Phenoxyethanol / | |
| | Methylparaben / | Methylparaben / | |
| | Ethylparaben / | Ethylparaben / | |
| | Propylparaben / | Propylparaben / | |
| | Butylparaben / | Butylparaben / | |
| | Isobutylparaben | Isobutylparaben | 0,500 |
| AV 50 TSI | Titanium Dioxide / | Titanium Dioxide / | |
| | Isocetyl Stearoyl Stearate | Isocetyl Stearoyl Stearate | |
| | / Avocado Oil | / Avocado Oil | |
| | Unsaponifiables / | Unsaponifiables / | |
| | Triethoxy Caprylylsilane | Triethoxy Caprylylsilane | |
| | / Water | / Aqua | 3,000 |
| BYO-12 (gelb) | Iron Oxide / Isopropyl | CI 77492 / Isoporopyl | |
| | Titanium Triisostearate | Titanium Triisostearate | 0,300 |
| BRO-12 (rot) | Iron Oxide / Isopropyl | CI 77491 / Isoporopyl | |
| | Titanium Triisostearate | Titanium Triisostearate | 0,300 |
| BBO-12 (schwarz) | Iron Oxide / Isopropyl | CI 77491 / Isoporopyl | |
| | Titanium Triisostearate | Titanium Triisostearate | 0,100 |
| GMS/MM3 | Mica / Magnesium | Mica / Magnesium | |
| | Myrista | Myrista | 0,100 |
| Propylenglykol | Propylene Glycol | Propylene Glycol | 5,000 |
| Panthenol | Panthenol | Panthenol | 1,000 |
| Rooibos | Rooibus (Leaf) Extract | Aspalathus Linearis | 0,100 |
| Ascorbinsäure | Ascorbic Acid | Ascorbic Acid | 0,200 |
| Natriumbisulfit- | Sodium Hydrogensulfite | Sodium Hydrogensulfite | |
| Lösung 39 % | | | 0,200 |
| Citronensäure | Citric Acid | Citric Acid | 0,100 |
| Wasser | Water | Aqua | ad 100 |

### Beispiel 4: Lippenstift mit folgender Zusammensetzung

| Handelsname | CTFA | INCI | Anteil in % |
|---|---|---|---|
| Rizinusöl | Castor Oil | Ricinus Communis | 46,700 |
| Bienenwachs | Beeswax | Cera Alba | 10,000 |
| Eutanol G | Octyldodecanol | Octyldodecanol | 10,000 |
| Candelillawachs | Candelilla Wax | Candelilla Cera | 6,500 |
| Tegosoft SH | Stearyl Heptanoate | Stearyl Heptanoate | 6,500 |
| Ozokerit | Ozokerite | Ozokerite | 5,000 |
| Jojobaöl | Jojoba Oil | Buxus Chinensis | 5,000 |
| Isopropyllanoat | Isopropyl Lanoate | Isopropyl Lanoate | 5,000 |
| Carnaubawachs | Carnauba Wax | Carnauba | 3,500 |
| Vitamin-E-Acetat | Tocopheryl Acetate | Tocopheryl Acetate | 0,500 |
| Bisabolol | Bisabolol | Bisabolol | 0,200 |
| Rooibos | Rooibus (Leaf) Extract | Aspalathus Linearis | 0,100 |
| Aroma | Aroma | Aroma | 1,000 |
| | | | 100,000 |

## Patentansprüche

1. Rooibos-Extrakt,
**dadurch gekennzeichnet, dass** er einen Aspalathingehalt von mindestens 10 Gew.-% sowie einen Chlorophyllgehalt von weniger als 0,4 Gew.-% aufweist und hergestellt ist durch eine Lösemittelextraktion mit den Schritten
- Bereitstellen getrockneter und zerkleinerter, unfermentierter Rooibos-Rohware,
- Extrahieren der bereitgestellten Rohware mit einem Extraktionsmittel bestehend aus einer Mischung aus Ethanol und Wasser für eine vorbestimmte Extraktionsdauer,
- Filtrieren des Extraktes,
- Einengen des filtrierten Extraktes unter reduziertem Druck bis zur Trockene,
- Mahlen des getrockneten Extraktes,
- Waschen des gemahlenen Extraktes mit einem Lösemittel für eine vorbestimmte Zeitdauer,
- Filtrieren des Extrakt/Lösemittel-Gemisches und Trocknen des resultierenden Filterkuchens unter reduziertem Druck bis zum Erreichen eines vorgegebenen Restgehalts an Lösemittel.

2. Rooibos-Extrakt nach Anspruch 1,
**dadurch gekennzeichnet, dass** der Aspalathingehalt mehr als 15 Gew.-% beträgt.

3. Rooibos-Extrakt nach Anspruch 1 oder 2,
**dadurch gekennzeichnet, dass** der Chlorophyllgehalt weniger als 0,2 Gew.-% beträgt.

4. Verfahren zur Herstellung eines Rooibos-Extraktes,
**gekennzeichnet durch** die Schritte
- Bereitstellen getrockneter und zerkleinerter, unfermentierter Rooibos-Rohware,
- Extrahieren der bereitgestellten Rohware mit einem Extraktionsmittel bestehend aus einer Mischung aus Ethanol und Wasser für eine vorbestimmte Extraktionsdauer,
- Filtrieren des Extraktes,
- Einengen des filtrierten Extraktes unter reduziertem Druck bis zur Trockene,
- Mahlen des getrockneten Extraktes,
- Waschen des gemahlenen Extraktes mit einem Lösemittel für eine vorbestimmte Zeitdauer,
- Filtrieren des Extrakt/Lösemittel-Gemisches und Trocknen des resultierenden Filterkuchens unter reduziertem Druck bis zum Erreichen eines vorgegebenen Restgehalts an Lösemittel.

5. Verfahren nach Anspruch 4,
**dadurch gekennzeichnet, dass** der Feuchtegehalt der bereitgestellten Rooibos-Rohware 4% oder kleiner ist.

6. Verfahren nach Anspruch 4 oder 5,
**dadurch gekennzeichnet, dass** das Extraktionsmittel aus Ethanol und Wasser im Verhältnis 80:20 besteht.

7. Verfahren nach Anspruch 6,
**dadurch gekennzeichnet, dass** das Verhältnis Rohware zu Extraktionsmittel 1:5 beträgt.

8. Verfahren nach Anspruch 7,
**dadurch gekennzeichnet, dass** der Schritt des Extrahierens bei bei einer Temperatur von 15 bis 35 °C erfolgt.

9. Verfahren nach Anspruch 8,
**dadurch gekennzeichnet, dass** die vorbestimmte Extraktionsdauer 1,5 bis 3 Stunden beträgt.

10. Verfahren nach einem der Ansprüche 4 bis 9,
**dadurch gekennzeichnet, dass** der Schritt des Einengens des filtrierten Extraktes bei einem Druck von weniger als 100 mbar, vorzugsweise bei einem Druck von 30 oder weniger mbar erfolgt.

11. Verfahren nach Anspruch 10,
**dadurch gekennzeichnet, dass** der Schritt des Einengens des filtrierten Extraktes bei einer Temperatur von maximal 40 °C erfolgt.

12. Verfahren nach einem der Ansprüche 4 bis 11,
**dadurch gekennzeichnet, dass** der Schritt des Waschens des gemahlenen Extraktes mit einem Lösemittel bei einem Verhältnis Extrakt zu Lösemittel von 1:5 erfolgt.

13. Verfahren nach Anspruch 12,
**dadurch gekennzeichnet, dass** der Schritt des Waschens des gemahlenen Extraktes mit einem Lösemittel für eine Zeitdauer von 1,5 bis 3 Stunden erfolgt.

14. Verfahren nach einem der Ansprüche 4 bis 13,
**dadurch gekennzeichnet, dass** der vorgegebene Restgehalt an Lösemittel 20 ppm oder weniger beträgt.

## Claims

1. Rooibos extract,
**characterised in that** it has an aspalathin content of at least 10 wt% as well as a chlorophyll content of less than 0.4 wt% and is prepared by a solvent extraction comprising the steps of:
- providing dried and comminuted, unfermented rooibos raw material,
- extracting the supplied raw material with an extracting agent consisting of a mixture of ethanol and water for a predetermined extraction time,
- filtering the extract,
- concentrating the filtered extract to dryness by evaporation under reduced pressure,
- grinding the dried extract,
- washing the ground extract with a solvent for a predetermined time,
- filtering off the extract/solvent mixture and drying the resultant filter cake under reduced pressure until a predetermined residual content of solvent is obtained.

2. Rooibos extract according to claim 1,
**characterised in that** the aspalathin content is more than 15 wt%.

3. Rooibos extract according to claim 1 or 2,
**characterised in that** the chlorophyll content is less than 0.2 wt%.

4. Process for the preparation of a rooibos extract,
**characterised by** the following steps:
- providing dried and comminuted, unfermented rooibos raw material,
- extracting the supplied raw material with an extracting agent consisting of a mixture of ethanol and water for a predetermined extraction time,
- filtering the extract,
- concentrating the filtered extract to dryness by evaporation under reduced pressure,
- grinding the dried extract,
- washing the ground extract with a solvent for a predetermined time,
- filtering off the extract/solvent mixture and drying the resultant filter cake under reduced pressure until a predetermined residual content of solvent is obtained.

5. Process according to claim 4,
**characterised in that** the moisture content of the supplied rooibos raw material is 4% or less.

6. Process according to claim 4 or 5,
**characterised in that** the extracting agent consists of ethanol and water in a ratio of 80:20.

7. Process according to claim 6,
**characterised in that** the ratio of raw material to extracting agent is 1:5.

8. Process according to claim 7,
**characterised in that** the extraction step is carried out at a temperature of 15° to 35°C.

9. Process according to claim 8,
**characterised in that** the predetermined extraction time is 1.5 to 3 hours.

10. Process according to one of claims 4 to 9,
**characterised in that** the step of concentrating the filtered extract by evaporation is carried out at a pressure of less than 100 mbar, preferably at a pressure of 30 mbar or less.

11. Process according to claim 10,
**characterised in that** the step of concentrating the filtered extract by evaporation is carried out at a temperature of at most 40°C.

12. Process according to one of claims 4 to 11,
**characterised in that** the step of washing the ground extract is carried out with a solvent in a ratio of extract to solvent of 1:5.

13. Process according to claim 12,
**characterised in that** the step of washing the ground extract is carried out with a solvent for a time of 1.5 to 3 hours.

14. Process according to one of claims 4 to 13,
**characterised in that** the predetermined residual content of solvent is 20 ppm or less.

## Revendications

1. Extrait de rooibos,
**caractérisé en ce que** ledit extrait présente une teneur en aspalathine d'au moins 10% en poids ainsi qu'une teneur en chlorophylle de moins de 0,4% en poids et est obtenu par extraction par solvant à l'aide des étapes suivantes :
- mise à disposition du rooibos comme matière brute, non fermentée, réduite en morceaux et séchée,
- extraction de la matière brute mise à disposition à l'aide d'un agent d'extraction se composant d'un mélange d'éthanol et d'eau pour une durée d'extraction prédéfinie,
- filtration de l'extrait,
- concentration de l'extrait filtré sous pression réduite jusqu'au séchage de celui-ci
- mouture de l'extrait séché,
- lavage de l'extrait moulu à l'aide d'un solvant pour une durée prédéfinie
- filtration du mélange extrait/solvant et séchage sous pression réduite du gâteau de filtration ainsi obtenu jusqu'à obtention d'une teneur résiduelle prédéfinie en solvant.

2. Extrait de rooibos selon la revendication 1,
**caractérisé en ce que** la teneur en aspalathine est supérieure à 15% en poids.

3. Extrait de rooibos selon la revendication 1 ou 2,
**caractérisé en ce que** la teneur en chlorophylle est inférieure à 0,2% en poids.

4. Procédé pour produire un extrait de rooibos,
**caractérisé par** les étapes suivantes :
- mise à disposition du rooibos comme matière brute non fermentée, réduite en morceaux et séchée,
- extraction de la matière brute mise à disposition à l'aide d'un agent d'extraction se composant d'un mélange d'éthanol et d'eau pour une durée d'extraction prédéfinie,
- filtration de l'extrait,
- concentration de l'extrait filtré sous pression réduite jusqu'au séchage de celui-ci
- mouture de l'extrait séché,
- lavage de l'extrait moulu à l'aide d'un solvant pour une durée prédéfinie
- filtration du mélange extrait/solvant et séchage sous pression réduite du gâteau de filtration ainsi obtenu jusqu'à obtention d'une teneur résiduelle prédéfinie en solvant.

5. Procédé selon la revendication 4,
**caractérisé en ce que** la teneur en humidité du rooibos utilisé comme matière brute est égale ou inférieure à 4%.

6. Procédé selon la revendication 4 ou 5,
**caractérisé en ce que** l'agent d'extraction se compose d'éthanol et d'eau dans un rapport de 80/20.

7. Procédé selon la revendication 6,
**caractérisé en ce que** le rapport matière brute/agent d'extraction est de 1/5.

8. Procédé selon la revendication 7,
**caractérisé en ce que** l'étape de l'extraction est réalisée à une température comprise entre 15 et 35 °C.

9. Procédé selon la revendication 8,
**caractérisé en ce que** la durée d'extraction prédéfinie est de 1,5 à 3 heures.

10. Procédé selon l'une des revendications 4 à 9,
**caractérisé en ce que** l'étape de la concentration de l'extrait filtré est réalisée sous une pression inférieure à 100 mbar, de préférence sous une pression égale ou inférieure à 30 mbar.

11. Procédé selon la revendication 10,
**caractérisé en ce que** l'étape de la concentration de l'extrait filtré est réalisée à une température de 40 °C maximum.

12. Procédé selon l'une des revendications 4 à 11,
**caractérisé en ce que** l'étape du lavage de l'extrait moulu est réalisée à l'aide d'un solvant selon un rapport extrait/solvant de 1/5.

13. Procédé selon la revendication 12,
**caractérisé en ce que** l'étape du lavage de l'extrait moulu est réalisée à l'aide d'un solvant pour une durée comprise entre 1,5 et 3 heures.

14. Procédé selon l'une des revendications 4 à 13,
**caractérisé en ce que** la teneur résiduelle prédéfinie en solvant est égale ou inférieure à 20 ppm.
